# EUROPEAN PATENT SPECIFICATION

(11) **EP 0 835 266 B1**
(45) Date of publication and mention of the grant of the patent: **04.05.2005**
(21) Application number: 97916693.1
(22) Date of filing: 26.03.1997
(51) Int. Cl.: C07K 19/00, A61K 47/48, A61K 39/395, C07K 14/31

(54) **MODIFIED/CHIMERIC SUPERANTIGENS AND THEIR USE**
MODIFIZIERTE CHIMÄRE SUPERANTIGENE UND DEREN VERWENDUNG
SUPERANTIGENES MODIFIES/CHIMERIQUES ET UTILISATION DE CES DERNIERS

(30) Priority: 29.03.1996 SE 9601245; 12.08.1996 US 695692
(43) Date of publication of application: 15.04.1998
(73) Proprietor: Active Biotech AB, 220 07 Lund (SE)
(72) Inventor: ANTONSSON, Per, S-222 54 Lund (SE); HANSSON, Johan, S-226 57 Lund (SE); BJÖRK, Per, S-256 56 Helsingborg (SE); DOHLSTEN, Mikael, S-223 62 Lund (SE); KALLAND, Terje, I-20020 Arese (IT); ABRAHMSEN, Lars, S-168 58 Bromma (SE); FORSBERG, Göran, S-226 49 Lund (SE)
(74) Representative: Wiklund, Ingrid Helena
(86) International application number: PCT/SE1997/000537
(87) International publication number: WO 1997/036932

(56) References cited:
- EP-A- 0 682 039
- WO-A-96/01650
- US-A- 3 903 262
- US-A- 5 541 087
- J. EXP. MED., Volume 175, February 1992, JOHN W. KAPPLER et al., "Mutations Defining Functional Regions of the Superantigen Staphylococcal Enterotoxin B", pages 387-396.
- J. EXP. MED., Volume 177, February 1993, JOSEPH A. MOLLICK et al., "Localization of a Site on Bacterial Superantigens that Determines T Cell Receptor beta Chain Specificity", pages 283-293.
- J. EXP. MED., Volume 177, January 1993, KEITH R. HUDSON et al., "Two Adjacent Residues in Staphylococcal Enterotoxins A and E Determine T Cell Receptor Vbeta Specificity", pages 175-184.
- NATIONAL LIBRARY OF MEDICINE, File Medline, Medline Accession No. 95136241, IHLE J., "Antibody-Targeted Superantigens Induce Lysis of Major Histocompatibility Complex Class II-Negative T-Cell Leukemia Lines"; & CANCER RES., 1995 Feb. 1;55(3):623-8.
- NATIONAL LIBRARY OF MEDICINE, File Medline, Medline Accession No. 95385031, HOLZER U. et al., "Superantigen-Staphylococcal-Enterotoxin-A-Dependent and Antibody-Targeted Lysis of GD2-Positive Neuroblastoma Cells"; & CANCER IMMUNOL. IMMUNOTHER., 1995 Aug.; 41(2):129-36.
- NATIONAL LIBRARY OF MEDLINE, File Medline, Medline Accession No. 95347324, ABRAHMSEN L. et al., "Characterization of Two Distinct MHC Class II Binding Sites in the Superantigen Staphylococcal Enterotoxin A"; & EMBO J., 1995, Jul. 3;14(13):2979-86.
- DIALOG INFORMATION SERVICES, File 155, Medline, Dialog Accession No. 06620856, Medline Accession No. 91355698, GILLIES S.D. et al., "Antigen Binding and Biological Activities of Engineered Mutant Chimeric Antibodies with Human Tumor Specificities"; & HUM. ANTIBODIES HYBRIDOMAS, 1990, 1(1), p47-54.
- PROC. NATL. ACAD. SCI. U.S.A., Volume 90, January 1993, M. BETTER et al., "Potent Anti-CD5 Ricin A Chain Immunoconjugates from Bacterially Produced Fab' and F(ab')2", pages 457-461.
- DIALOG INFORMATION SERVICES, File 34, SciSearch, Dialog Accession No. 13524601, MIKOLAJCZYK S.D. et al., "High-Field, Site-Specific Coupling of N-Terminally Modified Beta-Lactamase to a Proteolytically Derived Single-Sulfhydryl Murine Fab'"; & BIOCONJUGATE CHEMISTRY, 1994, V5, N6, P636-646.

## Description

This application claims priority from Swedish Patent Application No 9601245-5, which was filed March 29, 1996, and from US application 08/695,692, which was filed August 12, 1996.

### Field of the invention

The present invention relates to functionally active modified superantigens which are wild-type superantigens (SA I) in which one or more amino acid residues have been substituted while maintaining superantigen function. In case one or more of the substituting residues (or a conserved amino acid residue thereof) occur in the corresponding positions in another wild-type superantigen (SA II), the modified superantigen is called a chimera (sometimes hybrid). Chimeric superantigens thus will contain part sequences/regions deriving from at least two different wild-type superantigens.

By the term "corresponding" is meant that residues, part sequences and regions replacing each other have functionally the same position in superantigens I and II so that substitution will lead to a chimeric form that is able to function as a superantigen.

The terminology grafted/grafting/graft is used in connection with parts of the full sequence of superantigen II that have replaced corresponding parts of superantigen I, even if only one single amino acid has been replaced.

Modified/chimeric superantigens also encompass functional superantigens modified in other ways, for instance modified by amino acid replacements in regions other than those related to the invention, conjugated to a target-seeking moiety including also fused forms when the moiety is a polypeptide/protein. The order for carrying out the modifications may vary. See below.

### SUPERANTIGENS

According to the very first definition (around 1988-1993), superantigens are bacterial or viral proteins capable of binding to MHC class II antigens without prior intracellular processing and activate T cells by binding to the β-chain variable region (Vβ) of the T cell receptor (TCR). The binding leads to a Vβ family restricted activation of a relatively large proportion/subset of T cells and lysis of MHC Class II expressing cells (superantigen dependent cell-mediated cytolysis = SDCC).

Well known wild-type superantigens according to the definition above are the staphylococcal enterotoxins (SEA, SEB, SEC1, SEC2, SED, SEE and SEH). Further examples are Toxic Shock Syndrome Toxin 1 (TSST-1, also of staphylococcal origin), Exfoliating Toxins (EXft), Streptococcal Pyrogenic Exotoxin A, B and C (SPE A, B and C), Mouse Mammary Tumor Virus proteins (MMTV), Streptococcal M proteins, Clostridial Perfringens Enterotoxin (CPET), mycoplasma arthritis superantigens etc. For a review of superantigens and their properties see Kotzin et al 1993.

During the early nineties it was discovered that activation and subsequent cell lysis could occur in a MHC class II independent manner in case the superantigen was conjugated with a target-seeking moiety capable of binding to a cell surface structure (Dohlsten et al WO9201470 and Abrahmsén et al WO9601650). Upon incubation of target cells (carrying the target structure for the target-seeking moiety) and effector cells (T cells) with the conjugates, the target cells become lysed (superantigen antibody dependent cell-mediated cytolysis = SADCC) without any requirement for class II expression. Accordingly the superantigen concept of today and used in the context of the present invention, if not otherwise specified, encompasses any compound (preferably of polypeptide structure) that is capable of binding to a cell surface structure (target structure) and to one or more polymorphic TCR chain, in particular the Vβ chain, thereby activating a subset of T cells expressing the specific TCR chain involved in the binding. The T cells then become cytotoxic for cells carrying the surface structure (target structure, target cells). Normally the activated subset of T cells constitutes about 1-20% of the total amount of T cells of an individual.

### BACKGROUND ART - STRUCTURAL AND FUNCTIONAL STUDIES UTILIZING MUTATED AND CHIMERIC SUPERANTIGENS.

Chimeric superantigens including point mutated forms have previously been described (Kappler et al WO 9314364, Kappler et al 1992; Grossman et al 1991; Hufnagle et al 1991; Hartwig et al 1993; Fraser et al 1993; Mollick et al 1993; Irwin et al 1992; Hudson et al 1993; and Blanco et al 1990). Mollick et al and Hudson et al show from studies of chimeras that the Vβ specificity of SEA and SEE resides in certain amino acid sequences present in the carboxy terminal region (i.e. amino acid residues 200, 206 and 207). In addition to the Vβ specificity, mainly depending on this region, Mollik et al also were able to show that for complete reconstitution of SEE like activity of SEA containing SEE grafts towards Vβ8, a fragment containing the N-terminal 70 amino acid residues from SEE was needed. This fragment contains parts of the SEE-like MHC class II α chain binding site and chimeric SEA/SEE molecules containing this part from SEE, inhibited binding of SEA to MHC class II DR1 in a SEE-like manner.

Recently SEE-SEA chimers involving an exchange of regions involved in binding to TCRVβ have been described (Lamphaer et al., J. Immunol. 156 (March 15, 1996) 2178-2185). A SEE superantigen Fab antibody fusion protein in which the SEE domains involved in the interaction with T cells have been replaced with the corresponding non-homologous SEA domains has been discussed at ABRF'96: Biomolecular Techniques, Holiday Inn Golden Gateway, San Francisco, Califonia March 30 - April 2, 1996 (Björk et al., M45).

### BACKGROUND ART - THERAPEUTIC USE OF SUPERANTIGENS

Non-conjugated superantigens have been suggested for therapy with curative effect presumably being accomplished through a general activation of the immune system (Kalland et al WO9104053; Terman et al WO9110680 and WO9324136; Newall et al 1991).

It has also been suggested to use modified superantigens conjugated to target-seeking moieties (Dohlsten et al WO9201470; Abrahmsén et al WO9601650).

This enabled a broader therapeutic use of T cell activation through Vβ. The conjugates studied so far have had a diminished class II affinity, which in turn has lead to a decrease of the severe systemic toxicity normally associated with the wild-type superantigens.

Terman et al (WO9110680; WO9324136) in side-sentences suggested cancer therapy with modified superantigens and superantigen fragments.

Kappler et al (WO9314634) have suggested to use non-conjugated superantigens (SEB) mutated to have lost their Vβ-binding or MHC Class II binding ability (in the context of vaccines and to neutralize toxic effects of superantigens). Abrahmsén et al (WO9601650) have suggested cancer therapy with conjugated superantigens having a modified, preferably decreased, ability to bind to Class II antigens. The modifications encompassed single mutations as well as construction of chimeras between different superantigens.

### THE PROBLEMS THAT HAVE BEEN THE OBJECTIVE TO SOLVE WITH THE PRESENT INVENTION.

The sera of human populations normally contain high titers of antibodies against superantigens. For the staphylococcal superantigens, for instance, the relative titers are TSST-1 > SEB > SEC1 > SE3 > SEC2 > SEA > SED > SEE. These relative titers indicate immunogenicity problems and problems with neutralizing antibodies in case SEs are administered parenterally. Based solely on these problems, SEE should be the preferred staphylococcal superantigen. In the context of work with fusion proteins, however, we have found that the ability for T cell MHC class II independent cytotoxicity, superantigen-antibody dependent cell cytotoxicity (SADCC), of SEE conjugates is poor. The anti-SE titers also indicate that there might be advantages in modifying a "high titer" superantigen to be more like a "low titer" superantigen.

### THE OBJECTIVES OF THE PRESENT INVENTION.

A first objective is to improve the previously known superantigens with respect to lowering their immunogenicity and reaction with neutralizing antibodies.

A second objective is to provide superantigens with less side effects when used as a drug.

A third objective is to provide improved superantigens that can be used as the active principle in the treatment of mammals suffering from cancers, autoimmune diseases, parasitic infestations, viral infections or other diseases associated with cells that on their surface express MHC class II antigens and/or structures that are specific for respective disease and bind to a target-seeking moiety incorporated into the superantigen.

### THE DISCOVERY THAT HAS RESULTED IN THE INVENTION

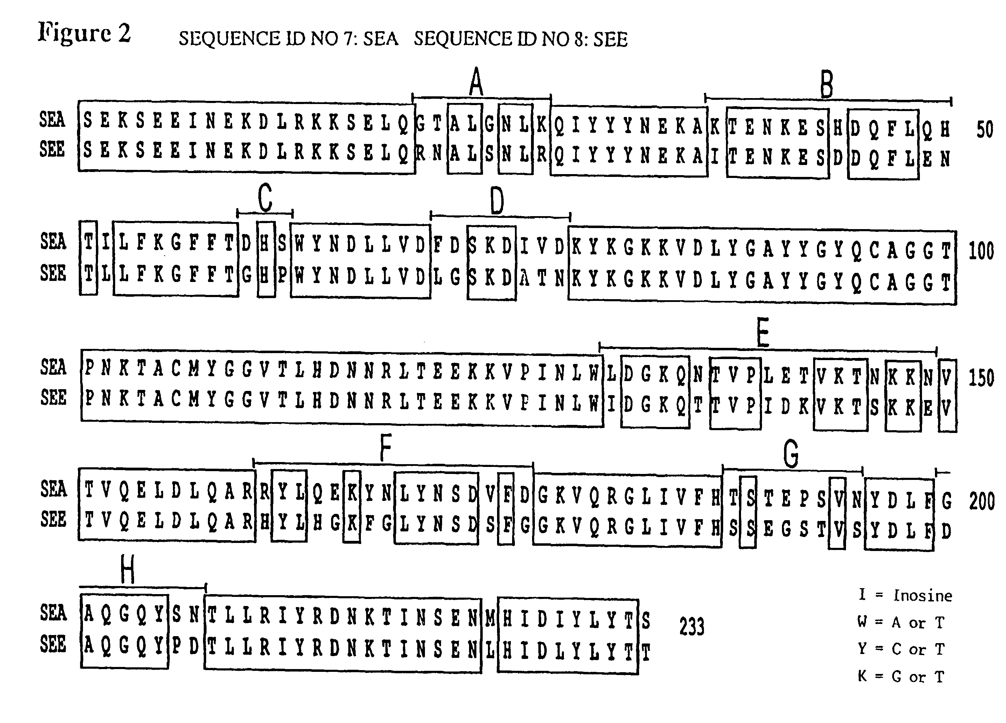

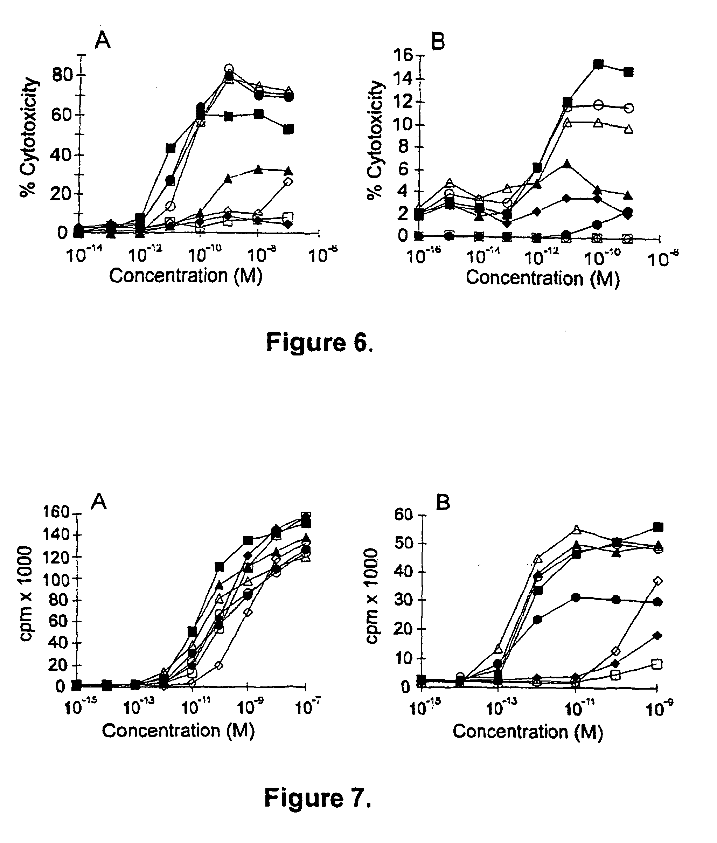

A sequence homology analyzis of SEA and SEE (Fig 2) reveals that the non-identical amino acid residues are concentrated to eight distinct regions. Outside these eight regions, making up to 34% of the sequence, the identity of the two SEs is 97%, with conserved amino acid substitutions accounting for the remaining differences. Four of these regions are structurally close to the two MHC class II binding sites (B: AA 37-50, D: 71-78, E: 136-149, and G 189-195), and are not likely to interact with the TCR. The additional four regions (A: AA 20-27, C: 60-62, F: 161-176 and H:200-207) are located on the edge of the molecule, in the vicinity of the putative TCR binding site, postulated to reside in the groove between the two subdomains. By grafting the individual regions (replacement of amino acid residues that differ), we have now found that the property of SEA-conjugates to induce a cytoxic response as well as potentiating proliferative response in the absence of MHC class II, resides in one region in the TCR binding domain of SEA. This Region (A) is transferable to SEE and has a great impact on activity in the absence of Class II, although limited effects on the Vβ specificity of the superantigen (Fig 6, Tab.2). All of the regions (A, C, F and H) seem to participate, directly or indirectly, in the interaction with the TCR manifested by an altered stimulatory effect on murine T-cell hybridomas (Tab. 2)

Due to the analogous mode of action it is conceivable that a similar structural separation of these TCRVβ binding properties is at hand also for superantigens analogous to SEA and SEE. The same may also apply within other types of superantigens, in which the binding structures are organised differently. Our discovery has enabled us to outline the construction of chimeric superantigens that potentially are of extremely great value as therapeutic agents.

### THE INVENTION

The first aspect of the invention is a method for the treatment of a disease in a mammal by activation of its immune system through administration of a therapeutically effective (immune activating) amount of a modified, preferably chimeric, superantigen. The mammal is preferably a human. The diseases in question are mostly associated with cells expressing on their surface a target structure binding to the superantigen. The target structure is in most cases different from the TCR epitope normally binding to superantigens. Binding to the target structure permits also binding to TCR and T cell activation. Illustrative examples are MHC class II antigens and other cell surface structures that may be expressed on cells associated with the courses of diseases. Illustrative diseases are malignant tumors including any type of cancers (such as carcinoma, sarcoma, melanoma, lymphoma etc.), viral infections, parasitic infestations and autoimmune diseases. The cancer to be treated may be located to the colon, breast, cervix, kidney, stomach, small intestines, duodenum, prostate, testis, skin, lung, liver, pancreas, skeleton etc including also metastasis at various locations. The inventive active agent is also applicable to so called multi-drug resistant forms of cancers. The cells expressing the target structure may also be cells that in some way control or regulate the development of the disease to be treated.

The characteristic feature of the method is that one employs a modified superantigen in which one or more amino acid residues in a region (region I) providing for binding to a subset of T cells via a polymorphic TCR chain, in particular TCRVβ, in a wild-type superantigen (SA I) has been replaced with a respective amino acid residue retaining superantigen activity to the so modified superantigen. The presently preferred embodiments refer to a chimeric superantigen in which one or more amino acid residues in a region (region I) of a first wild-type superantigen (SA I) have been replaced with the corresponding one or more amino acid residues in a corresponding region (region II) of a second wild-type superantigen (SA II). The regions I and II differ with respect to amino acid sequences. The superantigens I and II have been selected so that the regions I and II can replace each other without killing the superantigen function. In this context one has to account for the fact that a certain region I alone may not be interchangeable with the corresponding region of another wild-type superantigen although when interchanged together with other regions determining TCR binding and T cell activation, the result becomes a functional active superantigen. The regions concerned normally comprise less than 20 residues, in particular for superantigens analogous to SEA. The replacing amino acid residue thus is different from the replaced residue, and conceivably includes also conserved substitutions and other amino acid substitutions leading to funtionally active modified superantigens allowing binding to TCRVβ and activation of a subset of T cells. This means that the inventively modified superantigens in its broadest sense encompass any modified superantigen in which one or more amino acids in the aforementioned regions have been functionally replaced.

The term "conserved substitution" refers to replacement of an amino acid residue by a chemically similar residue, e.g. a hydrophobic residue for a different hydrophobic residue, a charged residue for a different but similarly charged residue etc.

As superantigens I, II etc, the staphylococcal enterotoxins, in particular those that coordinate zinc, were at the priority date preferred, i.e. SEA, SEE, SED and possibly also SEH.

The regions involved may have either of the above-mentioned functions (see the heading "The Discovery that has resulted in the Invention" and the Experimental Part):
1. A great impact on the superantigen activity as such and a limited effect on the TCR specificity, in particular on Vβ specificity. For SEA-type superantigens this means region A (amino acid positions 20-27).
2. A profound effect on the specificity with respect to binding to polymorphic TCR chains, such as the Vβ chain. For SEA-type of superantigens this means regions C (amino acid positions 60-62), F (amino acid positions 110-126) and H (amino acid positions 200-207).

For SEA-like superantigens this means one or more of the substitutions (applied to grafting from SEA to SEE; SEE/A chimeras):
Region A: R20G, N21T, S24G, R27K
Region C: G60D, P62S
Region F: H111R, H114Q, G115Y, F117Y, G118N, S124V, G126D
Region H: D200G, P206S, D207N
At the priority date it was preferred to carry out all substitutions for each region. For other superantigens, analogous substitutions between corresponding positions/regions could conceivably also be carried out.

Typically one could start from one first superantigen, like SEE and SED, and then replace one or more of its unique Vβ binding regions with the corresponding region(s) of a second superantigen (e.g. SEA), the first and second superantigens preferably being selected so that the antibody titer in normal human sera for the first superantigen is lower than for the second superantigen. For SEA and SEE chimeras, the best modes correspond to the chimeras SEE/A-A, SEE/A-AH, and SEA/E-BDEG, with absolute preference for SEE/A-A. See the experimental part and the figures.

Together with the regions A, C, F and H also amino acid residues at other parts can be exchanged. One type of exchange is to reduce the class II binding ability, because this property is associated with common side effects encountered in superantigen therapy (general immune activation with concomitant systemic release of tumor necrosis factor (TNF) and interferon-γ). For superantigens such as SEA, SED and SEE, positions that are important for the ability to coordinate zinc ions may preferably be changed, i.e. positions 225 and 227, for instance in SEA mutation H225A and in particular D227A will have a positive impact on reducing toxic side effects (see Abrahmsén et al WO9601650 and Fraser et al 1993).

Other substitution may be performed althroughout the molecule as long as they do not destroy the superantigen function, for instance conserved substitutions, in particular outside regions involved in the binding to class II and TCR. A change in the DNA sequence for altering the MHC class II binding or any other change on the DNA level may be carried out either before or after the change in regions providing for binding to TCR. These other types of modifications can equally well have been introduced prior to the amino acid replacement in Region I. In the context of the present invention, the concept of using a "wild-type superantigen" at the start of the modification according to the claims thus primarily refers to the wild-type amino acid sequence in region I outside of which prior modifications may have taken place.

Construction of chimeric and mutated superantigens can be carried out according to techniques well-known in the art. The switch from a region specific for one superantigen to the corresponding region in another superantigen is done on the genomic level and may be accomplished by replacing a complete sequence or by point mutations of those specific bases that are required to end up in the desired amino acid sequence. See for instance the experimental part and also the prior art references cited above. The term "mutation" comprises replacing, inserting or removing one or more amino acid residues by modifying the DNA sequence coding for the protein to be mutated.

The superantigen to be used in the inventive method can be a non-conjugated superantigen modified as described above, i.e. a modified superantigen lacking a specifically attached target-seeking moiety but with a pronounced ability to bind to both MHC class II antigens and a subset of T cells via TCR. More preferably the modified superantigen, preferably a chimeric superantigen, is conjugated to a target-seeking moiety. In the latter case the preferred variants are fusions between the target-seeking moiety and the modified superantigen. The conjugates as such are novel and are a separate aspect of the invention.

The structures of the inventive conjugates are analogous to earlier known antibody-superantigen conjugates (Dohlsten et al WO9201470; Abrahmsén et al WO9601650), i.e. the conjugates often comply with the formula:

T-B-SA(m)

where T represents the target-seeking moiety, SA(m) the modified, preferably chimeric, superantigen as defined above, and B is a covalent bridge linking T and SA(m) together. T may in principle contain further superantigen moieties (SA(m)), and SA(m) further target-seeking moieties, although in the preferred conjugates there is only one target-seeking moiety and one modified superantigen moiety as defined above.

T can in principle be any structure that is able to bind to a cell surface structure, preferably a disease specific structure. The structure against which T is directed is usually different from (a) the Vβ chain epitope to which SA(m) binds, and (b) the MHC class II epitopes to which superantigens bind. The target-seeking moiety is primarily selected among interleukins (e.g. interleukin-2), hormones, antibodies including antigen binding fragments of antibodies, growth factors etc. See for instance Woodworth, Preclinical and Clinical development of Cytokine toxins presented at the conference "Molecular approaches to cancer Immunotherapy", Ashville, North Carolina, November 7-11, 1993.

At the priority date, it was preferred that T was an antibody (full length antibody, Fab, F(ab)₂, Fv, single chain antibody and any other antigen binding antibody fragment), with particular emphasis for antibody active fragments (such as Fab), directed towards the so called C242 epitope (Lindholm et al WO9301303) or more preferably towards the binding epitope for the lung cancer specific 5T4 antibody (Stern et al WO8907947). This, however, does not exclude that other cancer specific antibodies may function equally well or even better. The term "antibody" comprises monoclonal as well as polyclonal variants, with preference for monoclonal preparations.

T may also be directed towards unique structures on more or less healthy cells that regulate or control the development of a disease.

The bridge B may be selected as previously described (Dohlsten et al WO9201470; and Abrahmsén et al WO9601650), i.e. B shall preferably be hydrophilic and exhibit one or more structure(s) selected among amide, thioether, disulphide etc. The most prominent bridges are those obtained by recombinant techniques, i.e. the conjugation takes place at the genomic level. In such cases oligopeptide bridges containing hydrophilic amino acid residues, such as Gln, Ser, Gly, Glu, Pro, His and Arg are preferred. Particularly preferred Bs are peptide bridges consisting of 1-10 amino acid residues, with absolute preferences for 3-7 amino acid residues. A typical bridge is the tripeptide GlyGlyPro, SEQ ID NO 1.

The manufacture of the novel inventive conjugates may be carried out in principle according to two main routes: 1. Recombinant techniques and 2. Chemical linking of a target-seeking moiety T to a modified, preferably chimeric, superantigen (SA(m)) as defined above. These methods are well recognized for the ordinary skilled worker and comprise a large number of variants.

Chemical linking of a modified non-conjugated superantigen to a target-seeking moiety T often utilizes functional groups (e.g. primary amino groups or carboxy groups) that are present in many positions in the compounds. It follows that the final product will contain a mixture of conjugate molecules differing in linking positions, as well as hetero- and homo-conjugates.

For recombinant conjugates (fusion proteins) the obtained conjugate substance will be uniform with respect to the linking position. Either the amino terminal of the chimeric superantigen is linked to the carboxy terminal of the target-seeking moiety or vice versa. For antibodies, such as intact antibodies and antigen-binding fragments (Fab, Fv, single chain antibodies etc), either the light or the heavy chain may be utilized for fusion. At present time recombinant conjugates are preferred, with utmost preference for Fab fragments and linking of the amino terminal of the chimeric superantigen to the first constant domain of the heavy antibody chain (CH1), without exclusion of the analogous linking to the light chain or to the VH and VL domain that also may give quite good results.

The main host cell for large scale recombinant production of the inventive modified superantigens (fused forms as well as non-conjugated forms) is E. coli. This host provides for in principle two routes: intracellular production and secretion. The latter variant is preferred because it offers purification of correctly folded proteins from the periplasma and from the culture medium. The above does not exclude that it is possible to produce active conjugates also in other host cells, e.g. eukaryotic cells, such as yeast or mammalian cells.

### PHARMACEUTICAL COMPOSITIONS, DOSAGE AND ROUTES OF ADMINISTRATION.

A third aspect of the instant invention is pharmaceutical compositions containing the inventive modified, preferably chimeric, superantigens as defined above (both conjugated and non-conjugated forms). The compositions contemplated are known in the field, except that now they contain the instant inventive superantigen. Thus, the compositions may be in the form of a lyophilized particulate material, a sterile or aseptically produced solution, a tablet, an ampoule etc. Vehicles such as water (preferably buffered to a physiologically acceptable pH value by for instance PBS) or other inert solid or liquid material may be present. In general terms the compositions are prepared by the conjugate being mixed with, dissolved in, bound to, or otherwise combined with one or more water-soluble or water-insoluble aqueous or nonaqueous vehicles, if necessary together with suitable additives and adjuvants. It is imperative that the vehicles and conditions must not adversely affect the activity of the modified superantigen.

Normally the inventive superantigen will be sold and administered in predispensed dosages, each one containing an effective amount of the conjugate that, based on the result now presented, is believed to be within the range of 10ng - 50 mg, such as within 10 ng - 1 mg or within 10 µg - 50 mg. The exact dosage will vary from case to case depending on the patient's weight and age, route of administration, type of disease, target-seeking moiety, superantigen, linkage (-B-) etc.

The administration routes will be those commonly contemplated within the field, i.e. a target cell killing effective amount or therapeutically active amount of a superantigen modified according to the invention is brought into contact with the target cells. For the indications specified above this mostly means parenteral administration, such as injection or infusion (subcutaneously, intravenously, intraarterial, intramuscularly, intraperitoneal) to a mammal, such as a human being. The modified, preferably chimeric, superantigens contemplated may be administered locally or systemically.

By the term "target killing effective amount" is contemplated that the amount is effective in activating and directing T cells to destroy target cells.

The preferred administration route at the priority date is the same as contemplated for the superantigen conjugates according to Dohlsten et al WO9201470 and Abrahmsén et al WO9601650. This means 1-5 hours' intravenous infusion (preferably 4 hours) per day combined with a fever-reducing agent (paracetamol). The administration is to be repeated during some days, for instance 4 days, with care consideration taken for the risk of boostering antibodies directed towards the conjugate.

The inventive superantigens may be administered either as the main therapy or in preferred modes as adjuvant therapy in connection with surgery or other drugs.

In the context of therapy we have found that antibody preparations that are pure with respect to non-covalently associated heavy and light antibody chains provide advantages over preparations that contains antibodies in which the chains are linked together via cystine linkages. Accordingly a fourth aspect of the invention is the therapeutic use of an antibody preparation, in particular an Fab preparation, in which the cysteine residues linking the chains together have been replaced by an amino acid not permitting disulfide formation, for instance serine. The most preferred antibody specificities for this aspect of the invention were at the priority date the C242 mab (Lindholm et al., WO9301302) and the 5T4 mab as defined in the references cited above. In the preferred variants one of the antibody chains is fused to a superantigen that is capable of activating a subset of T cells in a Vβ specific manner as described above. The superantigen may be a wild-type, a chimera, or a point-mutated version (and combination thereof) as described above or by Dohlsten et al WO9201470 or by Abrahmsén et al WO9601650. This aspect of the invention also comprises pharmaceutical compositions as described above, but containing an antibody preparation as defined for this aspect of the invention instead of a chimeric superantigen.

At the priority date it was preferred to use the Fab fragment 5T4 antibody (Stern et al, WO8907947) in combination with the SEE/A-A chimer with the mutation D227A. The preferred Fab fragment was mutated in both chains in the position providing interchain disulfide linkage (cys to ser). In order to increase the yield of the antibody/fusion protein when produced in E coli, mutations were also carried out in the Vkappa chain at certain positions. See the experimental part.

### MATERIALS AND METHODS

### Construction of SEA/SEE chimeric genes

Construction of SEA/SEE chimeras were made using the polymerase chain reaction (PCR) based method, sequence overlap extension (Horton et al). PCR reactions were performed with UlTma (Perkin-Elmer) according to manufactures recommendations. PCR produced fragments were cloned in PCR-script (Stratagene, USA) and sequenced to verify the correct sequence. The chimeric superantigen genes were then subcloned in the expression vector pKP889 (Abrahmsén et al 1995), fusing the SE constructs to the heavy chain portion to the Fab fragment of the murine monoclonal antibody C215. The SEA and SEE recombinant fusion proteins were produced as full length polypeptides in accordance with the consensus sequence for signal peptide cleavage (von Heijne 1986)

### Protein expression and purification

The *Escherichia coli* K12 strain UL635 was used for expression of the Fab-SE fusion proteins and the SEA mutants as described earlier (Abrahmsén et al 1995). Fab-SE fusion proteins were harvested by centrifugation at 5000 g and the supernatant fractions were subjected to purification on protein G Sepharose (Pharmacia Biotech AB, Uppsala, Sweden) as earlier described (Abrahmsén et al 1995). The purity of the affinity purified Fab-SE variants were >90% pure when analyzed by SDS-PAGE.

### Cells

The human B-cell lymphoma cell line Raji and human colon carcinoma Colo 205 were cultured in complete R-medium (RPMI-1640 supplemented with 10% fetal calf serum (Gibco BRL, Life Technologies, Ltd. Paisley Scotland) 1 mM glutamine; HyClone Europe, Ltd. Cramlington, 5x10⁻⁵ M β-mercaptoethanol; ICN Biomedicals INC. Costa Mesa CA, 0.1 M NaHCO₃; Seromed Biochrome, 1x10⁻² M Hepes buffer; HyClone Europe, Ltd. Cramlington., 0,1 mg/ml gentamycine; Biological Industries Kibbutz Beit Haemek Israel, 1x10⁻³ M sodium pyruvate; HyClone Europe, Ltd. Cramlington). CHO cells transfected with human C215 and CD80 molecules were cultivated in complete R-medium supplemented with 0.5 mg/ml Geniticin (G418) Gibco BRL, Life Technologies, Ltd. Paisly Scotland). Peripheral blood mononuclear cells (PBM) were prepared from heparinized blood from normal donors. The cells were isolated by density centrifugation over Ficoll-Paque as previously described (Dohlsten et al 1991). Human T lymphocytes were purified to homogenicity by positive selection using MiniMACS columns in conjunction with magnetic beads coated with monoclonal antibodies specific for human CD4 and CD8 (Miltenyi Biotec GmbH, Germany ) according to the manufacturers specifications. Human SEA and SEE reactive cell lines were generated as previously described (Dohlsten et al 1994). Human TCR Vβ22 expressing cell line was generated from a primary stimulated SEA reactive cell line using positive selection with magnetic Dynabeads (Dynal A.S., Norway) coated with TCR Vβ22 specific monoclonal antibody (Immunotech, France). Enriched cells contained > 95 % TCR Vβ22⁺ T cells as determined by flow cytometry (data not shown). Murine T-cell hybridomas (I1B3, 2B4 and 11.40) were generated as described (Fleury et al 1991).

### Cytotoxicity assay

Cytotoxicity was measured in a standard ⁵¹Cr release assay after 4 or 6 hours as previously described (Dohlsten et al 1991). Human Colo205 or Raji cells were used as target cells. The effector cells, either SEA or SEE reactive human T cell lines or TCR Vβ22 cell lines, were added at an effector to target ratio of 30:1. ⁵¹Cr-labeled target cells were used in the cytotoxicity assays at 2500 cells/200 ml complete medium in V-bottomed microtiter wells. C215Fab-SEA/E hybrids were added at various concentrations as indicated and ⁵¹Cr release was measured in a g-counter. The percentage specific cytotoxicity was calculated as 100x[(c.p.m. experimental release - c.p.m. background release)/( c.p.m. total release - c.p.m. background release)].

### Lymphocyte proliferation assays

To measure proliferation 10⁵ human T cell responders were incubated at 37°C with 10⁴ irradiated (20.000 Rad) stimulator cells in 200 ml complete medium in U-shaped 96-well microtitre plates with varying amounts of C215Fab-SEA/E hybrids for 72 hours. Proliferation was estimated by incorporation of [³H]-thymidine as described (Dohlsten et al 1988).

### Analysis of Fab-SAg induced IL-2 production.

Murine T-T hybridoma cells (10⁵) were incubated in 200 ml complete R-medium with C215Fab-SEA/E chimeric proteins in the presence of 2x10⁴ Raji stimulator cells. After 48 hours, supernatants were harvested and analyzed for presence of murine IL-2. Briefly, cytokine content was analyzed using rat anti-mouse cytokine mAb as catcher antibodies. Purified rat anti-mouse IL-2, biotin-labeled rat anti-mouse IL-2, rIL-2 was purchased from PharMingen (San Diego, CA). Biotin-labeled anti-cytokine mAb, Vectastain ABC kit (Vector Laboratories, CA) and peroxidase substate kit (Bio-Rad Laboratories, CA) were used for detection of cytokines. The absorbance was determined in a ImmunoReader NJ2000 (InterMed Roskilde, Denmark) at 405 or 450 nm.

### Mutation of 5T4 Fab

### Construction of a vector for expression of 5T4Fab-SEA in E. coli.

The Fv-encoding portions of 5T4 were cloned from the 5T4 hybridoma, obtained from Dr Peter Stern (Stern et al., WO8907947). In more detail: cDNA was made from the mRNA, regions of the entire variable domains and parts of the signal sequences as well as the first constant domain of the heavy chain and the constant domain of the light chain were amplified by PCR. The oligonucleotides and were used for the heavy chain, resulting in a 553 bp fragment, while the oligonucleotides and were used for the light chain, yielding a 724 bp fragment. For each chain three separate clones were sequenced and found to be identical. DNA fragments suitable for insertion into the expression vector (ref) were obtained in a second PCR step. In order to assemble a Fab-expression plasmid, the variable regions of 5T4 were fused to sequences coding for constant regions from the murine IgG1/k antibody C242 mab (Lindholm et al, WO9301302). A region coding for a superantigen derived from staphylococcal enterotoxin A (SEA) was fused after the heavy chain. The verified sequence for the Vkappa chain antibody framework for the 5T4 antibody is given in the results.

### Mutagenesis of 5T4

Seven amino acid replacements were introduced in the regions coding for the antibody framework. These were Phe10Ser, Thr45Lys, Ile63Ser, Tyr67Ser, Phe73Leu, Thr77Ser and Leu78Val. Similarly, the Cys residues in either chain involved in the interdomain disulfide bond were replaced by serine residues resulting in the mutations Cys458Ser in the heavy chain and Cys214Ser in the light chain. The mutations were introduced using PCR-based mutagenesis and the DNA sequence obtained was confirmed using sequencing.

### Fermentor expression and purification of 5T4Fab-SEA.

The expression plasmid contains the kanamycin resistance gene and a lacUV5-promoter that may be induced with IPTG. The fusion proteins were purified from the clarified culture medium using protein G Sepharose and SP-Sepharose (Pharmacia Biotec, Uppsala, Sweden) and formulated in citrate buffer using Sephadex G-25, essentially as described. Characterization using SDS-PAGE, reverse phase HPLC and mass spectrometry showed that the purified fusion protein was more than 95 % pure and had the correct molecular mass.

### RESULTS: SUPERANTIGEN MODIFICATIONS.

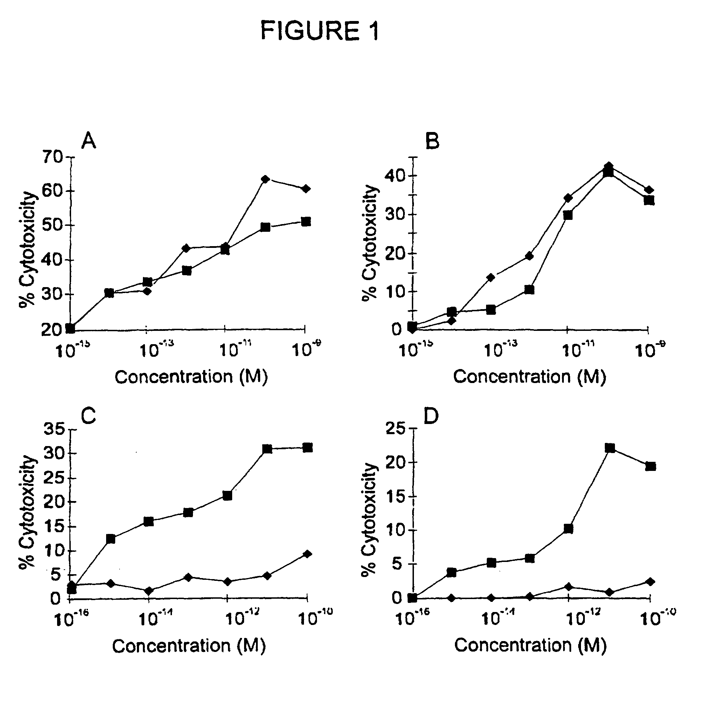

The superantigen dependent cellular cytotoxicity (SDCC) of C215Fab-SEA and of C215Fab-SEE against MHC class II⁺ Raji cells, was analyzed using SEA- and SEE-reactive human T cells as effector cell lines. Despite the difference in Vβ specificity between SEA and SEE both superantigens exhibited induction of comparable degree of cytotoxicity with both effector cell lines (Fig. 1). To discriminate between effects of MHC class II presentation and direct effects of SEA and SEE in TCR recognition, they were examined in superantigen-antibody dependent cellular cytotoxicity (SADCC) against C215 expressing Colo205 cells. In this assay the Fab moiety directs the fusion protein to C215-expressing target cells and results in the presentation of fused SE molecules to cytotoxic T-cells (CTL) independent of MHC class II molecules (Dohlsten et al 1994). Despite >80% amino acid sequence identity between SEA and SEE the TCR interaction of SEA and SEE displays qualitative differences in this type of assay. The C215Fab-SEA fusion protein retains its ability to direct SEA and SEE reactive CTL against the MHC class II⁻ target cells (FIG 1) while C215Fab-SEE fails to induce cytotoxicity of the, MHC class II⁻ target cells, neither with SEA nor with SEE reactive CTL (FIG 1).

It has previously been reported by other investigators that the differences in Vβ specificity between SEA and SEE primarily relates to a three amino acid difference in the loop preceding and in the irregular a5 helix (Irwin et al 1992, Hudson et al 1993, Fraser et al 1993, and Mollick et al 1993). The difference in respect to TCR interaction reported in this investigation is not related to altered TCR Vβ specificity since the ability of C215Fab-SEA to induce MHC class II independent cytotoxicity is not restricted to SEA reactive CTL but is also seen with SEE reactive CTL.

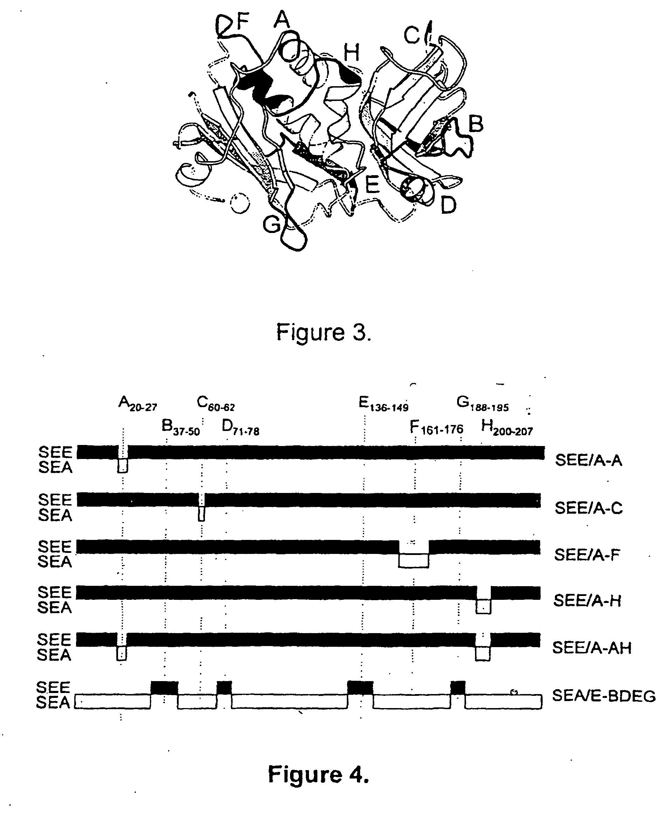

Sequence homology analysis of SEA and SEE (Fig. 2) reveals that the non-identical amino acid residues are concentrated to eight distinct regions. Outside these eight regions, making up to 34% of the sequence, the identity of the two SE's is 97%, with conserved amino acid substitutions accounting for the remaining differences. Four of the non-homologous regions are structurally close to the two MHC class II binding sites (B, D, E and G), and are not likely to interact with the TCR (Fig. 3). The additional four regions (A: AA 20-27, C: 60-62, F: 161-176 and H: 200-207) are located on the edge of the molecule (Fig. 3), in the vicinity of the TCR binding site, located in the groove between the two subdomains (Kappler et al 1992). To investigate the qualitative difference in TCR recognition between SEA and SEE we made hybrid proteins by grafting the regions from SEA to SEE as single region chimeras (SEE/A-A, -C, -F, H) as double region hybrids (SEE/A-AH) and by grafting the regions located in the vicinity of the MHC class II binding sites on SEE to SEA (SEA/E-BDEG) (Fig 4). All of the chimeric SEs were expressed as C215Fab fusion proteins to be able to detect differences with respect to their activity in the absence of MHC class II.

### The SEA/E hybrid proteins in fusion with the C215Fab moiety displays difference in Fab targeted cytotoxic assays.

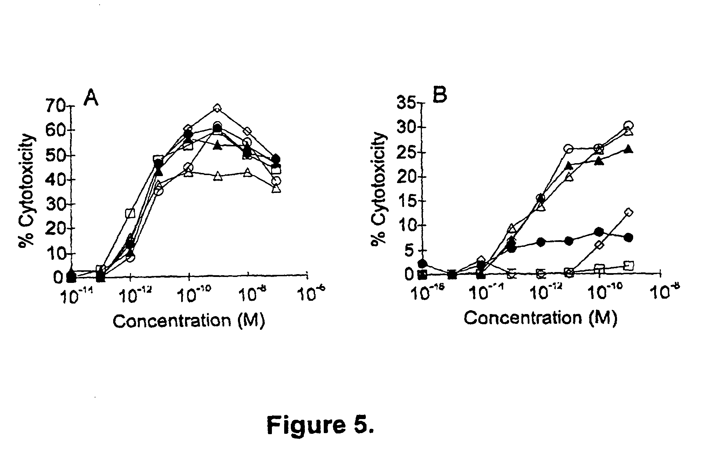

The SDCC activity of C215Fab-SEE/A hybrid proteins against MHC class II⁺ Raji cells were analyzed using SEA-reactive human T cells as effectors. The EC₅₀ values of all C215Fab-SE hybrids as well as the C215Fab-SEAwt and -SEEwt falls in the margin of errors (e.g. 10⁻¹²-10⁻¹¹ M, Fig 5). The only detectable difference is a slightly reduced plateau for the C215Fab-SEE/A-AH hybrid, indicating a loss of responding T cells. On the ther hand in SADCC experiments where the cytotoxicity is directed towards MHC class II⁻/C215⁺ Colo 205 cell line, only C215Fab-SEE/A-A, C215Fab-SEE/A-AH and C215Fab-SEA/E-BDEG induced comparable cytotoxicity as the C215Fab-SEAwt (Fig 5). The C215Fab-SEE/A-F hybrid is able to induce C215 targeted cytotoxicity at higher concentrations (EC₅₀ >10⁻¹⁰ M). Although the C215Fab-SEE/A-H hybrid is able to induce C215 targeted cytotoxicity with similar half maximal concentration as C215Fab-SEAwt (e.g. EC₅₀ 10⁻¹³ M), the absolute level of cytotoxicity is strongly reduced (Fig 5). This difference could be a consequence of a restricted Vβ specificity of the C215Fab-SEE/A-H while the ability of inducing C215 targeted cytotoxicity prevails in the responding T cell sub-population. To further investigate this notion we prepared human Vβ22 oligoclonal CTL line. Human Vβ22 are analogous to murine Vβ3 in the respect that it is a SEA non SEE specific Vβ family. It has previously been shown (Mollick et al 1993) that the major contribution of SEA and SEE Vβ is primarily residing in the three amino acid difference between SEA and SEE in region H (AA 200-207). In SDCC assays against MHC class II⁺ Raji targets, using the Vβ22 oligoclonal CTL line as effectors, only hybrids containing the SEA-H region are able to give C215Fab-SEAwt-like response (e.g. C215Fab-SEE/A-H, C215Fab-SEE/-AH and C215Fab-SEA/E-BDEG, Fig. 6). The C215Fab-SEE/A-A hybrid, that was able to induce a full SDCC response with whole CTL populations as effectors is in this assay strongly reduced both in half maximal concentration and in the plateau (Fig. 6). When the cytotoxicity of the Vβ22 CTL is directed towards the MHC class II⁻/C215⁺ Colo 205 cell line only hybrids containing both SEA-A and SEA-H (e.g. C215Fab-SEE/A-AH and C215Fab-SEA/E-BDEG) regions are able to induce a cytotoxic response, comparable to a C215Fab-SEAwt (Fig. 6). The hybrid containing only the SEA region A (C215Fab-SEE/A-A) induces a lower level of cytotoxicity with a comparable EC50 value. This indicates that the remaining activity seen with the C215Fab-SEE/A-H hybrid in SADCC with the whole T cell population as effectors is not a consequence of the hybrid induced response in restricted population of T cells. A more likely explanation for the observation is that the ability to induce a SADCC response of the C215Fab SE hybrid proteins is primarily residing in the SEA-A region with a minor contribution from the SEA-H and -F regions. There is no evidence that this quality is restricted to any subset of T cells in the combined SEA-SEE responding T cell population, since C215Fab SEA is able to induce the same response with as well with SEE reactive CTLs and C215Fab-SEE/A-A is able to fully to reconstitute the response seen with C215Fab-SEA.

### The SEA/E hybrid proteins in fusion with the C215Fab moiety displays difference in Fab targeted proliferation assays.

It has been previously shown that purified resting human T cells are induced to proliferate by presentation of C215Fab-SEA on a MHC class II⁻/C215⁺/CD80⁺ cell line (Lando et al 1993). The ability of C215Fab-SEA to induce MHC II independent proliferation is however markedly reduced with C215Fab-SEE (Tab. 1). To investigate if this difference in quality shows the same confinement to SEA region A, as was seen with SADCC, we investigated the proliferative capacity of C215Fab-SE hybrids, presented by either CHO-DR1⁺/CD80⁺ or CHO-C215⁺/CD80⁺ transfected cell lines, on purified resting human T cells. When presenting the Fab-SE conjugates on CHO-DR1⁺/CD80⁺ no differences between the different SE proteins were noted (data not shown). However grafts of SEA region A, C and H in SEE potentates the proliferative activity compared to C215Fab-SEE. The best results were obtained by grafting SEA regions A and H, indicating a important role for region A as was seen for the MHC class II independent cytotoxicity. By using a negative selection it is possible that the differences between Fab-SEA and -SEE would be more prominent.

### Vβ specificity of SE-hybrids

To further investigate if the C215Fab-SEA/SEE hybrid-fusion proteins were associated with a certain Vβ specificity we used SEA reactive murine T cell hybridomas expressing Vβ1, Vβ3 and V β11. It is obvious from the data obtained that all of the regions investigated, directly or indirectly, affects the interaction with the TCR. By grafting SEA regions C and F in C215Fab-SEE the activity towards the SEA and SEE cross reactive Vβ1 hybridoma I1B3 is destroyed. The same chimeras seems to have no or minor effects on the activity of Vβ3 and Vβ11 hybridomas (2.B4 and 11.40) in comparison with C215Fab-SEE. By grafting SEA region A in C215Fab-SEE the activity towards Vβ3 (2.B4) is enhanced by at least a factor 100, in comparison to C215Fab-SEE. More pronounced effects are seen with the same cell line by grafting SEA region H in C215Fab-SEE. This pronounced effect on the influence of Vβ3 specificity by SEA region H has also been noted by earlier investigations (Mollick et al 1993). The same chimera however (C215Fab-SEE/A-H), seems to reduce the activity towards the SEA/SEE cross reactive Vβ1 and Vβ11 hybridomas (I1B3 and 11.40) by a factor 10. In conclusion, the TCR interaction of SEA seems to involve all of the SEA-SEE, variable regions A, C, F and H.

### Seroreactivity

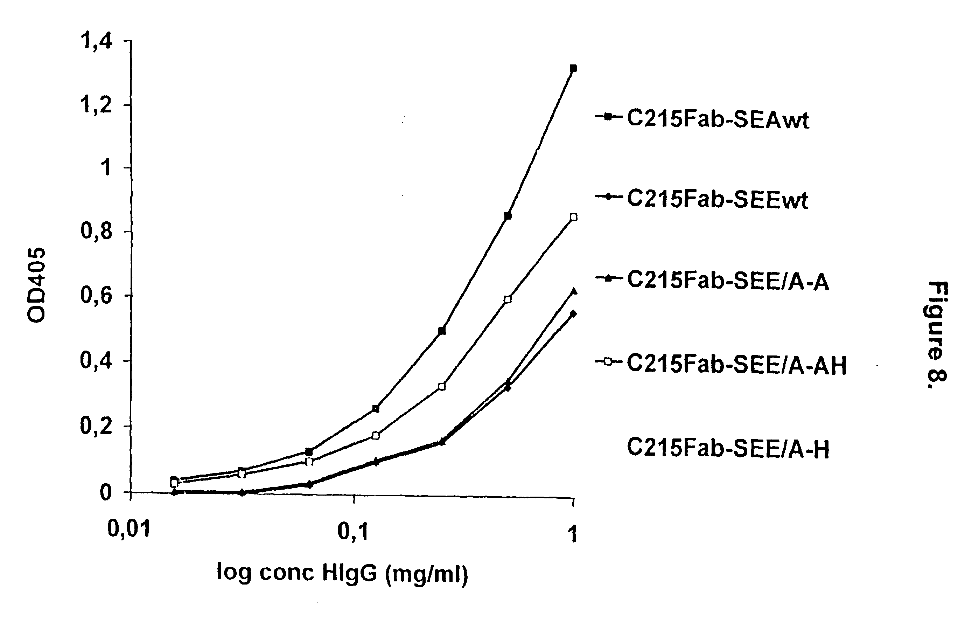

The seroreactivity in human serum samples towards the chimeric SEs was investigated both in pooled samples from different parts of the world as well as in individual serum samples. By grafting both SEA regions A and H in SEE we obtained an intermediate seroreactivity (Fig 8). A similar seroreactivity was also seen against the chimera C215Fab-SEE/A. However, single grafts of SEA region A in SEE (C215Fab-SEE/A-A) gave a C215Fab-SEE like seroreactivity, indicating that SEA region H is responsible for the remaining seroreactivity against C215Fab-SEE/A-AH. This indicates that the SEA region H is part of dominating antigenic epitope in SEA. The seroreactivity from pooled serum samples from other parts of the world (Japan and USA) as well as 14 individual samples from Sweden all confirms the same general pattern (data not shown).

### Results: Mutations of the Fab part of the fusion proteins.

### Expression of 5T4FabSEA-constructs

The production level in *E.coli* of 5T4Fab-SEA in the fermenter was found to be significantly lower than other Fab-superantigen constructs previously studied in our lab. Two types of modifications were therefore introduced to increase the production level. Firstly, seven different point mutations in the framework region of the light chain were introduced. These were Phe10Ser, Thr45Lys, Ile63Ser, Tyr67Ser, Phe73Leu, Thr77Ser and Leu78Val. Secondly, the cysteine residues making the disulfide bond connecting the heavy and the light chains were replaced by serine residues. The latter modification resulted in a three-fold increase and the 7 point mutations in an additional 12-fold increase in the production level. In addition to the significantly increased production level, removing the disulfide bond also gives a more homogenuous product since the possibility of the these reactive thiol groups to react with other thiol containing agents is excluded.

The modified 5T4 molecule was checked for affinity to its antigen as well as for biological activity in SADCC. No differences between the mutant form and the wildtype form could be detected in these assays.

The Cys/Ser mutation was also performed in the heavy and light chains of the Fab fragments of several other monoclonal antibodies. The products became homogenous and fully retained the antigen binding capability.

### Sequence of region of the antibody frame work for the 5T4 Vkappa chain:

Underlined sequences are CDRs. Bold-typed positions were mutated: Phe10Ser, Thr45Lys, Ile63Ser, Ile63Thr, Tyr67Ser, Phe73Leu, Thr77Ser, Leu78Val.

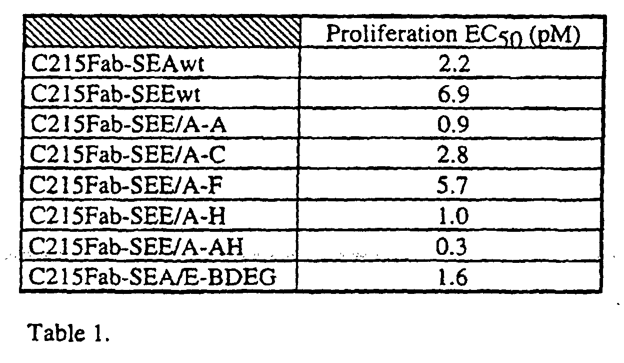

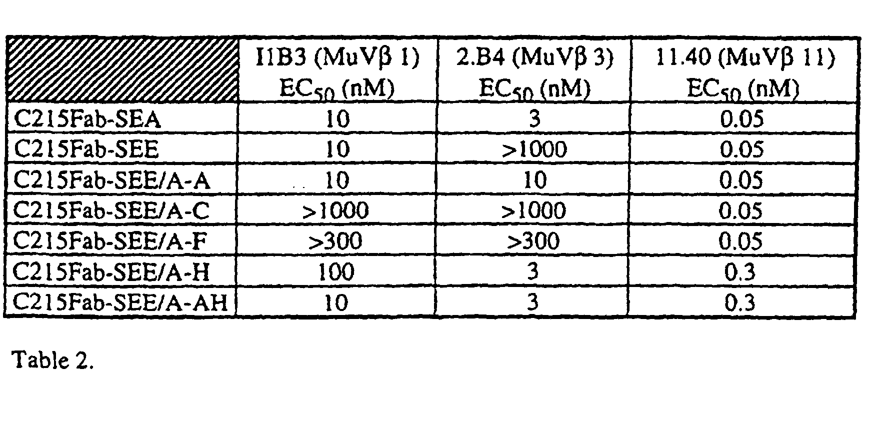

### Figure legends

### Figure 1. MHC class II dependent and independent cytotoxicity with human SEE and SEA CTL.

MHC class II dependent cellular cytotoxicity(A and B) and C215 dependent cellular cytotoxicity (C and D) with C215Fab-SEA (■) and C215Fab-SEE (◆) as effector molecules. Cytotoxicity was analyzed in a standard 4-h ⁵¹Cr release assay using a SEE-reactive human T-cell line (A and C) a SEA-reactive human T-cell line (B and D). Target cell lines were MHC class II⁺/ C215⁻ Raji (A and B) and MHC class II⁻/ C215⁺ Colo 205 (C and D). Data are from single assays that is representative of two (A and C) to five (B and D) independent experiments.

### Figure 2. Homology alignment of SEA and SEE.

SEA/SEE variable regions close to the TCR binding site (A, C, F and H) and variable regions close to the two MHC class II binding sites.

### Figure 3. Cartoon model of SEA.

Molscript model (Kraulis, 1991) of the SEA crystal (Schad et al. 1995). SEA/SEE variable regions close to the TCR binding site (A, C, F and H) and variable regions close to the two MHC class II binding sites. The zinc ion is a round ball.

### Figure 4. Schematic representation of chimeric SE molecules.

Stretches of SEA sequence are depressed. SEA/SEE variable regions are represented by A, B, C, D, E, F, G and H.

### Figure 5. MHC class II dependent and independent cytotoxicity with human SEA reactive CTL.

(A) MHC class II dependent cellular cytotoxicity and (B) C215 dependent cellular cytotoxicity of C215Fab-SEE/A-A (◆),C215Fab-SEE/A-C (□), C215Fab-SEE/A-F (◇), C215Fab-SEE/A-H (•), C215Fab-SEE/A-AH (Δ)and C215Fab-SEA/E-BDEG (○). Cytotoxicity was analyzed in standard 4-h ⁵¹Cr release assay using a SEA-reactive human T-cell line. Target cell lines were MHC class II⁺/ C215⁻ Raji (A) and MHC class II⁻/ C215⁺ Colo 205 (B). Data are from single assays that is representative of five independent experiments.

### Figure 6. MHC class II dependent and independent cytotoxicity with human Vb22⁺ CTL.

(A) MHC class II dependent cellular cytotoxicity and (B) C215 dependent cellular cytotoxicity C215Fab-SEA (■), C215Fab-SEE (◆), C215Fab-SEE/A-A (◆),C215Fab-SEE/A-C (□), C215Fab-SEE/A-F (◇), C215Fab-SEE/A-H (•), C215Fab-SEE/A-AH (Δ)and C215Fab-SEA/E-BDEG (○) as effector molecules. Cytotoxicity was analyzed in a standard 4h ⁵¹Cr release assay using a Vb22 selected SEA-reactive human T-cell line. Target cell lines were MHC class II⁺/ C215⁻ Raji ((A) and MHC class II⁻/ C215⁺ Colo 205 (B). Data are from single assays that is representative of two independent experiments.

### Figure 7. MHC class II dependent and independent proliferation (not in the priority applications).

Effects of Fab-SE hybrids on MHC class II dependent (A) and independent (B) T cell proliferation. Purified human T-cells were stimulated for 96h with C215Fab-SEA (■), C215Fab-SEE (◆), C215Fab-SEE/A-A (◆),C215Fab-SEE/A-C (□), C215Fab-SEE/A-F (◇), C215Fab-SEE/A-H (•), C215Fab-SEE/A-AH (Δ)and C215Fab-SEA/E-BDEG (○) presented on MHC class II⁺/C215⁻ CHO-DR4/C215 transfectants (A) and on MHC class II⁻/C215⁺ CHO-CD80/C215 transfectants (B). After 72h the cells were pulsed with [³H]-thymidine for 24h and incorporated label were measured and represented as half maximal concentration (EC₅₀). Data are from a single assays that is representative of two independent experiments.

### Figure 8. Seroreactivity in a human Ig pool.

Pool of >5000 sera from healthy donors in Southern Europe against C215Fab-SE fusion proteins. Serially diluted human Ig was allowed to interact for 1h at room temperature with C215Fab-SEAwt, C215Fab-SEEwt, C215Fab-SEE/A-A, C215Fab-SEE/A-H and FabSEE/A-AH. Immobilized to the micro titer plates at a concentration of 1 ng/well. Correction for C215Fab binding to serum proteins was made by subtracting the OD-value for C215Fab at each point. Each point represents the mean of duplicate samples. For further details see Materials and Methods.

**Table 1.** Purified human T-cells were stimulated for 96h with respective C215Fab-SE presented on MHC class II negative CHO-CD80/C215 transfectants. After 72h the cells were pulsed with ³H-thymidine for 24h and incorporated label was measured and represented as half maximal concentration (EC₅₀).

**Table 2.** Murine T cell hybridomas were stimulated for 48h with native or chimeric Fab conjugated superantigen. Activity was measured as IL-2 production and represented as half maximal concentration (EC₅₀).

### Work during the priority year.

In an attempt to minimize the toxicity of the superantigen chimer antibody fusion C242Fab-SEE/A-A, the chimer SEE/A-A has been mutated in Class II binding sites as described in WO9601650 and fused to C215Fab. The constructions are C215Fab-SEE/A-A-D227A, C215Fab-SEE/A-A-F47A/D227A, C215Fab-SEE/A-A-H187A/D227A, C215Fab-SEE/A-A-W130A/D227A, C215Fab-SEE/A-A-D70A/D227A, C215Fab-SEE/A-A-N50S/D227A, C215Fab-SEE/A-A-N50S/D70A/D227A, C215Fab-SEE/A-A-F47Y/D227A och C215Fab-SEE/A-A-D70R/D227A. All fusions have been tested for their ability to induce proliferation of human PBMC in order to identify mutants having a lowered activity compared to C215Fab-SEE/A-A-D227A. A lowered proliferative activity has been observed for C215Fab-SEE/A-A-F47A/D227A, C215Fab-SEE/A-A-F47Y/D227A och C215Fab-SEE/A-A-D70R/D227A. Some of the chimeric fusions have also been tested for antibody titer in human normal serum (C215Fab-SEE/A-A-D227/A, C215-FabSEE/A-A-D70A/D227A and C215Fab-SEE/A-A-D70R/D227A). A comparison was made with C215Fab-SEA-D227A and C215Fab-SEE-D227A. Relative C215FabSEA-D227A, the titer was much lower for each chimer tested. Relative C215Fab-SEE-D227A, the titer was slightly higher for each chimer tested.

This means replacements at positions corresponding to one or more, preferably two, of the positions 47, 50, 70, 130, 187, 227 as defined in sequence ID nos 7 and 8 in figure 2.

Because many varying and different embodiments may be made within the scope of the inventive concept herein taught, and because modifications may be made in the embodiments herein detailed in accordance with the descriptive requirements of the law, it is to be understood that the details herein are to be interpreted as illustrative and not in a limiting sense.

### REFERENCES

**Abrahmsén L et al** (1995) Characterization of two distinct MHC Class II binding sites in the superantigen staphylococcal enterotoxin A. EMBO J 14:2978-86.
**Abrahmsén et al** (1996) WO9601650 (patent application). A conjugate between a modified superantigen and a target-seeking compound and the use of the conjugate.
**Antonsson P et al** (1996) Staphylococcal enterotoxin A and E chimera with reduced seroreactivity and retained ability to target cytotoxic T cells for use in tumor therapy. ABRF '96: Biomolecular Techniques, Holiday Inn Golden Gateway, San Francisco, California. March 30-April 2, 1996.
**Dohlsten et al** (1988) Two subsets of human peripheral blood CD4+ T helper cells differing in the capacity to produce IL-2 and interferon-gamma can be defined by the Leu-18 and UCHL1 monoclonal antibodies. Eur J Immunol 18:1173-1178.
**Blanco et al** (1990) Mutants of staphylococcal toxic shock syndrome toxin 1: Mitogenicity and recognition by a neutralizing monoclonal antibody. Infect. Immun. 58 (1990) 3020-3028.
**Dohlsten M et al** (1994) Monoclonal antibody-superantigen fusion proteins: Tumor specific agents for T cell based tumor therapy. Proc Natl Acad Sci USA 91:8945-8949.
**Dohlsten M et al** (1991) Monoclonal antibody-targeted superantigens: A different class of anti-tumor agents. Proc Natl Acad Sci USA 88:9287-9291.
**Dohlsten et al** (1992) WO9201470 (patent application). Target specific antibody -superantigen conjugates and their preparation.
**Fleury S et al** (1991) Mutational analysis of the interaction between CD4 and class II MHC: class II antigens. Cell 66:1037-49.
**Fraser JD et al** (1993) Structural model of Staphylococcal enterotoxin A interactions with MHC class II antigens. In: Huber,BT Palmer, E (eds) Current Communications in Cell and Molecular Biology 7. Cold Spring Harbour Laboratory Press, Cold Spring Harbor, NY. pp 7-29.
**Grossman et al** (1991) Mutation of the disulfide loop in staphylococcal enterotoxin A. Consequences for T cell recognition. J Immunol 147:3274-3281.
**Hartwig UF et al** (1993) Mutations affecting MHC class II binding of the superantigen streptococcal erythrogenic toxin A. Int. Immunol. 5 (8) 869-875.
**Horton RM et al** (1990) Gene splicing by overlap extension: tailor-made genes using the polymerase chain reaction. Biotechniques 8:528-535
**Hudson et al** (1993) Two adjacent residues in staphylococcal enterotoxins A and E determine T cell receptor V beta specificity. J Exp Med 177:175-184.
**Hufnagle WO et al** (1991) The carboxyl-terminal region of staphylococcal enterotoxin type A is required for a fully active molecule. Infect Immun 59:2126-2134.
**Irwin MJ et al** (1992) Enterotoxin residues determining T-cell receptor Vb binding specificity. Nature 359:841-3
**Kalland et al** (1991) WO 9104053 (patent application). Pharmaceutical composition that makes cells expressing MHC Class II antigens targets for cytotoxic cells.
**Kappler JW et al** (1992) Mutations defining functional regions of the superantigen staphylococcal enterotoxin B. J Exp Med 175:387-396
**Kappler et al** (1993) WO9314634 (patent application). Protective effects of mutated superantigens.
**Kotzin BL et al** (1993) Superantigens and their potential role in human disease. Adv Immunol 54:99-166.
**Kraulis PJ** (1991) MOLSCRIPT: A program to produce both detailed and schematic plots of protein structures. J Appl Cryst 24:946-950.
**Lamphear JG et al** (1996) Residues near the amino and carboxy termini of staphylococcal enterotoxin E independently mediate TCRVβ-specific interactions. J Immunol 156: 2178-2185 (March 15, 1996)
**Lando PA et al** (1993) Co-stimulation with B7 and targeted superantigen is required for MHC class II-independent T-cell proliferation but not cytotoxicity. Immunology 80: 236-241.
**Lindholm et al** (1993) 9301302 (patent application). Tumor, carbohydrate antigen specific monoclonal antibody and cell line.
**Mollick JA et al** (1993) Localization of a site on bacterial superantigens that determines T cell receptor beta chain specificity. J Exp Med 177:283-293.
**Newall et al** (1991) In vivo T-cell activation by staphylococcal enterotoxin B prevents outgrowth of a malignant tumor. Proc Natl Acad Sci USA 88 1074-1078
**Schad EM et al** (1995) Crystal structure of the superantigen, Staphylococcal enterotoxin type A. EMBO J 14:3292-3301
**Stern et al** (1989) WO8907947 (patent application). Improvements relating to anigens.
**Terman et al** (1991) WO9110680 (patent application). Tumor killing effects of enterotoxins and related compounds.
**Terman et al** (1993) WO9324136 (patent application). Tumor killing effects of enterotoxins and related compounds.
**von Heijne, G** (1986). A new method for predicting signal sequence cleavage sites. Nucleic Acid Res, 14, 1483-1490.

## Claims

1. A conjugate between
a) a wild type superantigen that has been modified, and
b) a target seeking moiety;
**characterized in that** said modified superantigen is SEE, in which one or more amino acid residues in a region I, which region I is within the T Cell Receptor (TCR) binding site and determines binding to TCR Vβ chain and T cell activation, have been replaced by one or more amino acid residues from a corresponding position in SEA, such that said modified superantigen has improved ability to induce T cell cytotoxicity compared to SEE and has reduced seroreactivity compared to SEA, and
the modified superantigen further comprises a mutation that decreases its ability to bind to MHC Class II antigens as compared to either said wild-type superantigen.

2. The conjugate of claim 1 wherein said region I is region A of figure 2, consisting of amino acid residues 20 to 27 of SEQ ID NO 7.

3. The conjugate of claim 1 wherein said region I is region C of figure 2, consisting of amino acid residues 60 to 62 of SEQ ID NO 7.

4. The conjugate of claim 1 wherein said region I is region F of figure 2, consisting of amino acid residues 161 to 176 of SEQ ID NO 7.

5. The conjugate of claim 1 wherein said region I is region H of figure 2, consisting of amino acid residues 200 to 207 of SEQ ID NO 7.

6. The conjugate of claim 2 in which the following amino acid substitutions have been made: R20G, N21T, S24G and R27K where the positions are as defined in SEQ ID NO 7.

7. The conjugate of claim 6 further comprising a mutation at position 227 as defined in SEQ ID NO 7.

8. The conjugate of claim 7 wherein the mutation is D227A.

9. The conjugate of any one of claims 1-8 wherein said target seeking moiety is selected from the group consisting of an antigen binding antibody active fragment, an antigen binding antibody, a single-chain antibody, Fab; F(ab)2 and Fv.

10. Composition comprising a therapeutically effective amount of a conjugate between
a) a wild type superantigen that has been modified, and
b) a target seeking moiety;
for use in the treatment of a disease in a mammal by activation of the immune system of said mammal, the said conjugate being **characterized in that** said modified superantigen is SEE, in which one or more amino acid residues in a region I, which region I is within the T Cell Receptor (TCR) binding site and determines binding to TCR Vβ chain and T cell activation, have been replaced by one or more amino acid residues from a corresponding position in SEA, such that said modified superantigen has improved ability to induce T cell cytotoxicity compared to SEE and has reduced seroreactivity compared to SEA, and
the modified superantigen further comprises a mutation that decreases its ability to bind to MHC Class II antigens as compared to either said wild-type superantigen.

11. The composition of claim 10 wherein the disease is associated with cells expressing a surface target structure which binds to the superantigen fusion at a superantigen epitope structurally different from the TCR binding epitope and which binding allows for binding to TCR and T cell activation of the superantigen.

12. The composition of claim 10 or 11 wherein the disease is selected from the group consisting of cancers, viral infections, parasitic infestations and autoimmune diseases.

13. The composition of claim 12 wherein the disease is cancer.

14. The composition of claim 10 wherein said region I is region A of figure 2, consisting of amino acid residues 20 to 27 of SEQ ID NO 7.

15. The composition of claim 10 wherein said region I is region C of figure 2, consisting of amino acid residues 60 to 62 of SEQ ID NO 7.

16. The composition of claim 10 wherein said region I is region F of figure 2, consisting of amino acid residues 161 to 176 of SEQ ID NO 7.

17. The composition of claim 10 wherein said region I is region H of figure 2, consisting of amino acid residues 200 to 207 of SEQ ID NO 7.

18. The composition of claim 14 in which the following amino acid substitutions have been made: R20G, N21T, S24G and R27K where the positions are as defined in SEQ ID NO 7.

19. The composition of claim 18 further comprising a mutation at position 227 as defined in SEQ ID NO 7.

20. The composition of claim 19 wherein the mutation is D227A.

21. The composition of claims 10-20 wherein said target seeking moiety is selected from the group consisting of an antigen binding antibody active fragment, an antigen binding antibody, a single-chain antibody, Fab; F(ab)2 and Fv.

22. A conjugate according to any one of claims 1-9 for use as a medicament.

23. Use of a conjugate according to any one of claims 1-9, for the manufacturing of a medicament for treatment of a disease in a mammal by activation of the immune system.

24. Use according to claim 23, wherein the disease is associated with cells expressing a surface target structure which binds to the superantigen fusion at a superantigen epitope structurally different from the TCR binding epitope and which binding allows for binding to TCR and T cell activation of the superantigen.

25. Use according to claim 23 or 24, wherein the disease is selected from the group comprising cancers, viral infections, parasitic infestations and autoimmune diseases.

26. Use according to any one of claims 23-25, wherein the disease is cancer.

## Patentansprüche

1. Ein Konjugat zwischen
a) einem Wildtyp - Superantigen, das modifiziert wurde, und
b) einem zielgebenden Bereich;
**dadurch gekennzeichnet, dass** besagtes modifiziertes Superantigen SEE ist, bei dem ein oder mehrere Aminosäurereste in einer Region I, wobei sich diese Region I innerhalb der T-Zellrezeptor (TCR) Bindungsstelle befindet und die Bindung an die TCR Vβ - Kette und die T-Zellaktivierung bestimmt, durch einen oder mehrere Aminosäurereste aus einer korrespondierenden Position in SEA ersetzt wurden, so dass besagtes modifiziertes Superantigen eine verbesserte Fähigkeit zur Induzierung von T-Zellcytotoxizität im Vergleich zu SEE aufweist und eine verringerte Seroreaktivität im Vergleich zu SEA aufweist, und
das modifizierte Superantigen umfasst zusätzlich eine Mutation, die seine Fähigkeit zur Bindung an MHC Klasse II Antigene im Vergleich zu jedem der besagten Wildtyp-Superantigene verringert.

2. Das Konjugat von Anspruch 1, worin besagte Region I Region A von Figur 2 ist, die aus den Aminosäureresten 20 bis 27 von SEQ ID NO: 7 besteht.

3. Das Konjugat von Anspruch 1, worin besagte Region I Region C von Figur 2 ist, die aus den Aminosäureresten 60 bis 62 von SEQ ID NO: 7 besteht.

4. Das Konjugat von Anspruch 1, worin besagte Region I Region F von Figur 2 ist, die aus den Aminosäureresten 161 bis 176 von SEQ ID NO: 7 besteht.

5. Das Konjugat von Anspruch 1, worin besagte Region I Region H von Figur 2 ist, die aus den Aminosäureresten 200 bis 207 von SEQ ID NO: 7 besteht.

6. Das Konjugat von Anspruch 2, bei dem die folgenden Aminosäuresubstitutionen durchgeführt wurden: R20G, N21T, S24G und R27K, wobei die Positionen wie in SEQ ID NO: 7 definiert sind.

7. Das Konjugat von Anspruch 6, das zusätzlich eine Mutation in Position 227 wie in SEQ ID NO:7 definiert umfasst.

8. Das Konjugat von Anspruch 7, worin die Mutation D227A ist.

9. Das Konjugat von einem der Ansprüche 1 - 8, worin besagter zielgebender Bereich aus der Gruppe ausgewählt ist, die aus einem aktiven Antigen-bindenden Antikörperfragment, einem Antigen-bindenen Antikörper, einem einzelkettigen Antikörper, Fab, F(ab)₂ und Fv besteht.

10. Zusammensetzung, die eine therapeutisch wirksame Menge eines Konjugats zwischen
a) einem Wildtyp - Superantigen, das modifiziert wurde, und
b) einem zielgebenden Bereich;
zur Verwendung in der Behandlung einer Krankheit in einem Säugetier durch Aktivierung des Immunsystems von besagtem Säugetier umfasst, wobei besagtes Konjugat **dadurch gekennzeichnet ist, dass** besagtes modifiziertes Superantigen SEE ist, bei dem ein oder mehrere Aminosäurereste in einer Region I, wobei sich diese Region I innerhalb der T-Zellrezeptor (TCR) Bindungsstelle befindet und die Bindung an die TCR Vβ - Kette und die T-Zellaktivierung bestimmt, durch einen oder mehrere Aminosäurereste aus einer korrespondierenden Position in SEA ersetzt wurden, so dass besagtes modifiziertes Superantigen eine verbesserte Fähigkeit zur Induzierung von T-Zellcytotoxizität im Vergleich zu SEE aufweist und eine verringerte Seroreaktivität im Vergleich zu SEA aufweist, und
das modifizierte Superantigen umfasst zusätzlich eine Mutation, die seine Fähigkeit zur Bindung an MHC Klasse II Antigene im Vergleich zu jedem der besagten Wildtyp-Superantigene verringert.

11. Die Zusammensetzung von Anspruch 10, worin die Krankheit mit Zellen assoziiert ist, die eine Oberflächenzielstruktur exprimieren, die an die Superantigenfusion an einem Superantigenepitop bindet, das sich strukturell von dem TCR Bindungsepitop unterscheidet und dessen Bindung die Bindung an TCR und T-Zellaktivierung des Superantigens ermöglicht.

12. Die Zusammensetzung von Anspruch 10 oder 11, worin die Krankheit aus der Gruppe ausgewählt ist, die aus Krebsarten, viralen Infektionen, parasitärem Befall und Autoimmunerkrankungen besteht.

13. Die Zusammensetzung von Anspruch 12, worin die Krankheit Krebs ist.

14. Die Zusammensetzung von Anspruch 10, worin besagte Region I Region A von Figur 2 ist, die aus den Aminosäureresten 20 bis 27 von SEQ ID NO: 7 besteht.

15. Die Zusammensetzung von Anspruch 10, worin besagte Region I Region C von Figur 2 ist, die aus den Aminosäureresten 60 bis 62 von SEQ ID NO: 7 besteht.

16. Die Zusammensetzung von Anspruch 10, worin besagte Region I Region F von Figur 2 ist, die aus den Aminosäureresten 161 bis 176 von SEQ ID NO: 7 besteht.

17. Die Zusammensetzung von Anspruch 10, worin besagte Region I Region H von Figur 2 ist, die aus den Amniosäureresten 200 bis 207 von SEQ ID NO: 7 besteht.

18. Die Zusammensetzung von Anspruch 14, bei der die folgenden Aminosäuresubstitutionen durchgeführt wurden: R20G, N21T, S24G und R27K, wobei die Positionen wie in SEQ ID NO: 7 definiert sind.

19. Die Zusammensetzung von Anspruch 18, die zusätzlich eine Mutation in Position 227 wie in SEQ ID NO:7 definiert umfasst.

20. Die Zusammensetzung von Anspruch 19, worin die Mutation D227A ist.

21. Die Zusammensetzungen der Ansprüche 10 - 20, worin besagter zielgebender Bereich aus der Gruppe ausgewählt ist, die aus einem aktiven Antigen-bindenden Antikörperfragment, einem Antigen-bindenden Antikörper, einem einzelkettigen Antikörper, Fab, F(ab)₂ und Fv besteht.

22. Ein Konjugat gemäß einem der Ansprüche 1 - 9 zur Verwendung als Medikament.

23. Verwendung eines Konjugats gemäß einem der Ansprüche 1 - 9 zur Herstellung eines Medikaments zur Behandlung einer Krankheit in einem Säugetier durch Aktivierung des Immunsystems.

24. Verwendung gemäß Anspruch 23, worin die Krankheit mit Zellen assoziiert ist, die eine Oberflächenzielstruktur exprimieren, die an die Superantigenfusion an einem Superantigenepitop bindet, das sich strukturell von dem TCR Bindungsepitop unterscheidet und dessen Bindung die Bindung an TCR und T-Zellaktivierung des Superantigens ermöglicht.

25. Verwendung gemäß Anspruch 23 oder 24, worin die Krankheit aus der Gruppe ausgewählt ist, die aus Krebsarten, viralen Infektionen, parasitärem Befall und Autoimmunerkrankungen besteht.

26. Verwendung gemäß einem der Ansprüche 23 - 25, worin die Krankheit Krebs ist.

## Revendications

1. Conjugué entre
a) un superantigène de type sauvage qui a été modifié, et
b) une entité de recherche de cible ;
**caractérisé en ce que** ledit superantigène modifié est SEE, dans lequel un ou plusieurs résidus d'aminoacides dans une région I, laquelle région I est dans le site de liaison de récepteur de cellule T (TCR) et détermine la liaison à la chaîne Vβ de TCR et l'activation des cellules T, ont été remplacés par un ou plusieurs résidus d'aminoacides provenant d'une position correspondante dans SEA, de telle manière que ledit superantigène modifié a une aptitude améliorée à induire une cytotoxicité des cellules T par comparaison avec SEE et a une séroréactivité réduite par comparaison avec SEA, et
le superantigène modifié comprend en outre une mutation qui abaisse son aptitude à se lier à des antigènes du CMH de classe II par comparaison avec l'un ou l'autre superantigène de type sauvage.

2. Conjugué selon la revendication 1 où ladite région I est la région A de la figure 2, consistant en les résidus d'aminoacides 20 à 27 de SEQ ID NO 7.

3. Conjugué selon la revendication 1 où ladite région I est la région C de la figure 2, consistant en les résidus d'aminoacides 60 à 62 de SEQ ID NO 7.

4. Conjugué selon la revendication 1 où ladite région I est la région F de la figure 2, consistant en les résidus d'aminoacides 161 à 176 de SEQ ID NO 7.

5. Conjugué selon la revendication 1 où ladite région I est la région H de la figure 2, consistant en les résidus d'aminoacides 200 à 207 de SEQ ID NO 7.

6. Conjugué selon la revendication 2 où les substitutions d'aminoacides suivantes ont été faites : R20G, N21T, S24G et R27K où les positions sont définies comme dans SEQ ID NO 7.

7. Conjugué selon la revendication 6 comprenant en outre une mutation à la position 227 définie comme dans SEQ ID NO 7.

8. Conjugué selon la revendication 7 où la mutation est D227A.

9. Conjugué selon l'une quelconque des revendications 1-8 où ladite entité de recherche de cible est choisie dans le groupe consistant en un fragment actif d'anticorps liant des antigènes, un anticorps liant des antigènes, un anticorps à une seule chaîne, Fab, F(ab)2 et Fv.

10. Composition comprenant une quantité thérapeutiquement efficace d'un conjugué entre
c) un superantigène de type sauvage qui a été modifié, et
d) une entité de recherche de cible ;
destinée à être utilisée dans le traitement d'une maladie dans un mammifère par activation du système immunitaire dudit mammifère, ledit conjugué étant **caractérisé en ce que** ledit superantigène modifié est SEE, dans lequel un ou plusieurs résidus d'aminoacides dans une région I, laquelle région I est dans le site de liaison de récepteur de cellule T (TCR) et détermine la liaison à la chaîne Vβ de TCR et l'activation des cellules T, ont été remplacés par un ou plusieurs résidus d'aminoacides provenant d'une position correspondante dans SEA, de telle manière que ledit superantigène modifié a une aptitude améliorée à induire une cytotoxicité des cellules T par comparaison avec SEE et a une séroréactivité réduite par comparaison avec SEA, et
le superantigène modifié comprend en outre une mutation qui abaisse son aptitude à se lier à des antigènes du CMH de classe II par comparaison avec l'un ou l'autre superantigène de type sauvage.

11. Composition selon la revendication 10 où la maladie est associée avec des cellules exprimant une structure cible de surface qui se lie à la fusion de superantigène à un épitope du superantigène structuralement différent de l'épitope liant TCR et laquelle liaison permet la liaison du superantigène à TCR et l'activation de cellules T par le superantigène.

12. Composition selon la revendication 10 ou 11 où la maladie est choisie dans le groupe consistant en les cancers, les infections virales, les infestations parasitaires et les maladies auto-immunes.

13. Composition selon la revendication 12 où la maladie est un cancer.

14. Composition selon la revendication 10 où ladite région I est la région A de la figure 2, consistant en les résidus d'aminoacides 20 à 27 de SEQ ID NO 7.

15. Composition selon la revendication 10 où ladite région I est la région C de la figure 2, consistant en les résidus d'aminoacides 60 à 62 de SEQ ID NO 7.

16. Composition selon la revendication 10 où ladite région I est la région F de la figure 2, consistant en les résidus d'aminoacides 161 à 176 de SEQ ID NO 7.

17. Composition selon la revendication 10 où ladite région I est la région H de la figure 2, consistant en les résidus d'aminoacides 200 à 207 de SEQ ID NO 7.

18. Composition selon la revendication 14 où les substitutions d'aminoacides suivantes ont été faites : R20G, N21T, S24G et R27K où les positions sont définies comme dans SEQ ID NO 7.

19. Composition selon la revendication 18 comprenant en outre une mutation à la position 227 définie comme dans SEQ ID NO 7.

20. Composition selon la revendication 19 où la mutation est D227A.

21. Composition selon les revendications 10-20 où ladite entité de recherche de cible est choisie dans le groupe consistant en un fragment actif d'anticorps liant des antigènes, un anticorps liant des antigènes, un anticorps à une seule chaîne, Fab, F(ab)2 et Fv.

22. Conjugué selon l'une quelconque des revendications 1-9 destiné à être utilisé comme médicament.

23. Utilisation d'un conjugué selon l'une quelconque des revendications 1-9 pour la fabrication d'un médicament pour le traitement d'une maladie dans un mammifère par activation du système immunitaire.

24. Utilisation selon la revendication 23 où la maladie est associée avec des cellules exprimant une structure cible de surface qui se lie à la fusion de superantigène à un épitope du superantigène structuralement différent de l'épitope liant TCR et laquelle liaison permet la liaison du superantigène à TCR et l'activation de cellules T par le superantigène.

25. Utilisation selon la revendication 23 ou 24 où la maladie est choisie dans le groupe comprenant les cancers, les infections virales, les infestations parasitaires et les maladies auto-immunes.

26. Utilisation selon l'une quelconque des revendications 23-25 où la maladie est un cancer.


### SEQUENCE LISTING

(2) INFORMATION FOR SEQ ID NO:1:
   (i) SEQUENCE CHARACTERISTICS:
      (A) LENGTH: 3 amino acids
      (B) TYPE: amino acid
      (D) TOPOLOGY: linear
   (ii) MOLECULE TYPE: peptide
   (xi) SEQUENCE DESCRIPTION: SEQ ID No:1:
   Gly Gly Pro
   1
(3) INFORMATION FOR SEQ ID NO:2:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO:2:
   CAATTTTCTT GTCCACCTTG GTGC
(4) INFORMATION FOR SEQ ID NO:3:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:3:
   ACTAGTCGAC ATGGATGGAG CTITATCATI YTCTT
(5) INFORMATION FOR SEQ ID NO:4:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (xi) SEQUENCE DESCRIPTION: SEQ ID NO:4:
   ACTAGTCGAC ATGGGCITCA AGATGGAGTC ACAKWYYCWG G
(6) INFORMATION FOR SEQ ID NO:5:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO:5:
   GCGCCGTCTA GAATTAACAC TCATTCCTGT TGAA
(7) INFORMATION FOR SEQ ID NO:6:
   (i) SEQUENCE CHARACTERISTICS:
      (A) LENGTH: 107 amino acids
      (B) TYPE: amino acid
      (D) TOPOLOGY: linear
   (ii) MOLECULE TYPE: peptide
   (xi) SEQUENCE DESCRIPTION: SEQ ID NO:6:
   Asp Ala Val Met Thr Gln Thr Pro Thr Phe
   1               5                   10
   Leu Leu Val Ser Ala Gly Asp Arg Val Thr
                   15                  20
   Ile Thr Cys Lys Ala Ser Gln Ser Val Ser
                   25                  30
   Asn Asp Val Ala Trp Tyr Gln Gln Lys Pro
                   35                  40
   Gly Gln Ser Pro Thr Leu Leu Ile Ser Tyr
                   45                  50
   Thr Ser Ser Arg Tyr Ala Gly Val Pro Asp
                   55                  60
   Arg Phe Ile Gly Ser Gly Tyr Gly Thr Asp
                   65                  70
   Phe Thr Phe Thr Ile Ser Thr Leu Gln Ala
                   75                  80
   Glu Asp Leu Ala Val Tyr Phe Cys Gln Gln
                   85                  90
   Asp Tyr Asn Ser Pro Pro Thr Phe Gly Gly
                   95                  100
   Gly Thr Lys Leu Glu Ile Lys
                   105
(8) INFORMATION FOR SEQ ID NO:7:
   (i) SEQUENCE CHARACTERISTICS:
      (A) LENGTH: 233 amino acids
      (B) TYPE: amino acid
      (D) TOPOLOGY: linear
   (ii) MOLECULE TYPE: peptide
   (xi) SEQUENCE DESCRIPTION: SEQ ID NO:7:
   Ser Glu Lys Ser Glu Glu Ile Asn Glu Lys Asp Leu
                   5                   10
   Arg Lys Lys Ser Glu Leu Gln Gly Thr Ala Leu Gly
           15                  20
   Asn Leu Lys Gln Ile Tyr Tyr Tyr Asn Glu Lys Ala
   25                  30                  35
   Lys Thr Glu Asn Lys Glu Ser His Asp Gln Phe Leu
               40                  45
   Gln His Thr Ile Leu Phe Lys Gly Phe Phe Thr Asp
       50                  55                  60
   His Ser Trp Tyr Asn Asp Leu Leu Val Asp Phe Asp
                   65                  70
   Ser Lys Asp Ile Val Asp Lys Tyr Lys Gly Lys Lys
           75                  80
   Val Asp Leu Tyr Gly Ala Tyr Tyr Gly Tyr Gln Cys
   85                  90                  95
   Ala Gly Gly Thr Pro Asn Lys Thr Ala Cys Met Tyr
               100                 105
   Gly Gly Val Thr Leu His Asp Asn Asn Arg Leu Thr
       110                 115                 120
   Glu Glu Lys Lys Val Pro Ile Asn Leu Trp Leu Asp
                   125                 130
   Gly Lys Gln Asn Thr Val Pro Leu Glu Thr Val Lys
           135                 140
   Thr Asn Lys Lys Asn Val Thr Val Gln Glu Leu Asp
   145                 150                 155
   Leu Gln Ala Arg Arg Tyr Leu Gln Glu Lys Tyr Asn
               160                 165
   Leu Tyr Asn Ser Asp Val Phe Asp Gly Lys Val Gln
       170                 175                 180
   Arg Gly Leu Ile Val Phe His Thr Ser Thr Glu Pro
                   185                 190
   Ser Val Asn Tyr Asp Leu Phe Gly Ala Gln Gly Gln
           195                 200
   Tyr Ser Asn Thr Leu Leu Arg Ile Tyr Arg Asp Asn
   205                 210                 215
   Lys Thr Ile Asn Ser Glu Asn Met His Ile Asp Ile
               220                 225
   Tyr Leu Tyr Thr Ser
       230
(9) INFORMATION FOR SEQ ID NO:8:
   (i) SEQUENCE CHARACTERISTICS:
      (A) LENGTH: 233 amino acids
      (B) TYPE: amino acid
      (D) TOPOLOGY: linear
   (ii) MOLECULE TYPE: peptide
   (xi) SEQUENCE DESCRIPTION: SEQ ID NO:8:
   Ser Glu Lys Ser Glu Glu Ile Asn Glu Lys Asp Leu
                   5                   10
   Arg Lys Lys Ser Glu Leu Gln Arg Asn Ala Leu Ser
           15                  20
   Asn Leu Arg Gln Ile Tyr Tyr Tyr Asn Glu Lys Ala
   25                  30                  35
   Ile Thr Glu Asn Lys Glu Ser Asp Asp Gln Phe Leu
               40                  45
   Glu Asn Thr Leu Leu Phe Lys Gly Phe Phe Thr Gly
       50                  55                  60
   His Pro Trp Tyr Asn Asp Leu Leu Val Asp Leu Gly
                   65                  70
   Ser Lys Asp Ala Thr Asn Lys Tyr Lys Gly Lys Lys
           75                  80
   Val Asp Leu Tyr Gly Ala Tyr Tyr Gly Tyr Gln Cys
   85                  90                  95
   Ala Gly Gly Thr Pro Asn Lys Thr Ala Cys Met Tyr
               100                 105
   Gly Gly Val Thr Leu His Asp Asn Asn Arg Leu Thr
       110                 115                     120
   Glu Glu Lys Lys Val Pro Ile Asn Leu Trp Ile Asp
                   125                 130
   Gly Lys Gln Thr Thr Val Pro Ile Asp Lys Val Lys
           135                 140
   Thr Ser Lys Lys Glu Val Thr Val Gln Glu Leu Asp
   145                 150                 155
   Leu Gln Ala Arg His Tyr Leu His Gly Lys Phe Gly
               160                 165
   Leu Tyr Asn Ser Asp Ser Phe Gly Gly Lys Val Gln
       170                 175                 180
   Arg Gly Leu Ile Val Phe His Ser Ser Glu Gly Ser
                   185                 190
   Thr Val Ser Tyr Asp Leu Phe Asp Ala Gln Gly Gln
           195                 200
   Tyr Pro Asp Thr Leu Leu Arg Ile Tyr Arg Asp Asn
   205                 210                 215
   Lys Thr Ile Asn Ser Glu Asn Leu His Ile Asp Leu
               220                 225
   Tyr Leu Tyr Thr Thr
       230